# EUROPEAN PATENT APPLICATION

(11) **EP 4 252 745 A1**
(43) Date of publication of application: **04.10.2023**
(21) Application number: 21912448.4
(22) Date of filing: 11.01.2021
(51) Int. Cl.: A61K 9/16, A61K 47/69, A61K 47/10, A61K 47/34, A61K 36/60, A61K 31/05, A61K 31/352, A61P 29/00, A61P 35/00, A61P 35/02, A61P 25/16, A61P 25/08, A61P 25/28, A61P 1/16

(54) **WATER-SOLUBLE CANNABINOID FORMULATION AND PREPARATION METHOD THEREFOR**

(30) Priority: 29.12.2020 CN 202011596414
(71) Applicant: Hanyi Biotechnology (Beijing) Co., Ltd., Beijing 100020 (CN)
(72) Inventor: TAN, Xin, Beijing 100020 (CN); WANG, Shubin, Beijing 100020 (CN); FAN, Dekai, Beijing 100020 (CN)
(74) Representative: Murgitroyd & Company
(86) International application number: PCT/CN2021/071115
(87) International publication number: WO 2022/141665

(57) **Abstract**

Disclosed are a water-soluble cannabinoid formulation and a preparation method therefor. The water-soluble cannabinoid formulation comprises a cannabinoid, an amphiphilic high-molecular polymer and cyclodextrin, wherein the content of the cannabinoid in percentages by weight is 1-40%, the content of the amphiphilic high-molecular polymer in percentages by weight is 1-90%, and the content of the cyclodextrin in percentages by weight is 1-90%; and the preparation method comprises: (1) preparing a water-soluble composite solution from a cannabinoid, an amphiphilic high-molecular polymer and cyclodextrin; (2) drying the water-soluble composite solution obtained in step (1) to obtain water-soluble cannabinoid solid particles; and (3) preparing the cannabinoid solid particles obtained in step (2) into a water-soluble cannabinoid formulation. The water-soluble cannabinoid formulation has a high drug loading capacity, a low water consumption during preparation, a strong stability, a simple process, a low cost and a short inclusion time.

## Description

### TECHNICAL FIELD

The present invention relates to the field of pharmaceutical formulations, in particular to a water-soluble cannabinoid formulation and a preparation method therefor, and particularly to a water-soluble cannabinoid solid formulation and a preparation method therefor.

### BACKGROUND

Cyclodextrin (CD) is a generic name for a series of cyclic oligosaccharides produced by amylose under the action of cyclodextrin glucosyltransferase produced by Bacillus. An inclusion compound is a clathrate formed by embedding a (drug) molecule in a cavity structure of (formed by) another substance molecule (or a "molecular capsule"). An inclusion molecule having a cavity structure is called a host molecule, and an embedded molecule is called a guest molecule. Different types of host molecules can form inclusion compounds with different structures, such as tubular inclusion compounds, stratified inclusion compounds, cage-like inclusion compounds, single-molecule inclusion compounds, molecular sieve inclusion compounds, or high molecular inclusion compounds. A cyclodextrin inclusion compound is a clathrate formed by embedding a (drug) molecule in a cavity structure of a cyclodextrin molecule.

The cyclodextrin inclusion compound has the following characteristics: increasing the solubility and the dissolution rate of the drug; being able to make liquid drugs into powders and prevent volatilization; masking the unpleasant odor of the drug and reducing irritation; improving the stability of the drug; and preparing sustained/controlled-release formulations.

The preparation technology for the cyclodextrin inclusion compound includes the saturated aqueous solution method, solution-stirring, grinding, ultrasonication, lyophilization, spray drying, the liquid-liquid method, or the gas-liquid method.

A polymer micelle is a high molecular micelle which spontaneously forms a core-shell structure after an amphiphilic block copolymer is dissolved in water. The amphipathy is realized by a hydrophilic block and a hydrophobic block. The hydrophilic block includes polyethylene glycol (PEG), polyoxyethylene (PEO), and polyvinylpyrrolidone (PVP), and the hydrophobic block includes polypropylene, polyamino acid, polylactic acid, etc. Hydrophobic drugs may be embedded in a hydrophobic core, and the shell consisting of hydrophilic chain segments forms a hydration layer barrier to provide micelle stability. Two or more structurally different chain segments exist in a single linear copolymer molecule in a polymer micelle. A copolymer with a specific chemical structure and a specific molecular weight can be synthesized as desired, and a specific supramolecular ordered aggregate can be self-assembled in a solution.

The polymer micelle can increase the solubility of hardly soluble drugs in water, and can also enhance the penetration of the drugs to the vessel wall of tumor tissues by taking advantage of its extremely small particle size, thereby realizing the passive targeted drug release to tumors and prolonging the *in vivo* circulation time. Most of carriers of the polymer micelle are artificially synthesized, and they are biodegradable and thermodynamically stable in an aqueous medium.

The preparation technology of the polymer micelle includes direct dissolving, dialysis, oil-in-water emulsification, and solvent volatilization.

Cannabinoid is a lipid-soluble substance and is almost insoluble in water, which greatly restricts its development in the field of formulations. A cannabis formulation is mostly an oil solution, which is inconvenient to take, poor in stability, and low in absorption bioavailability.

The patent document CN 109953951 A discloses an oil-in-water nanoemulsion capable of increasing bioavailability of hardly soluble drug cannabidiol and a preparation method therefor. The patent document CN 110177543 A discloses that a water-soluble formulation of cannabinoid is obtained by first esterifying vitamin E succinate with polyethylene glycol 1000 (vitamin E TPGS) and then including the cannabinoid in micelles of the vitamin E TPGS.

The patent documents CN 109476625 A and CN 101998855 A both disclose that a water-soluble formulation of cannabinoid is obtained by including the cannabinoid in cyclodextrin.

Although including cannabinoid in nanoemulsion, nanocapsules, nanospheres, or cyclodextrin can improve the bioavailability of the cannabinoid, these known methods generally have the defects of low drug loading, poor stability, complex process, high cost, and the like.

### SUMMARY

To overcome the defects in the prior art, the present invention provides a water-soluble cannabinoid solid granule, which comprises a cannabinoid, an amphiphilic high molecular polymer, and cyclodextrin, wherein a content of the cannabinoid in percentages by weight is 1-40%, a content of the amphiphilic high molecular polymer in percentages by weight is 1-90%, and a content of the cyclodextrin in percentages by weight is 1-90%.

Specifically, the cannabinoid is selected from one of or a combination of two or more of pure products of CBD, CBDV, CBG, CBC, CBN, CBDB, CBE, CBL, and CBND; or specifically, the cannabinoid is a cannabis extract comprising one of or a combination of two or more of CBD, CBDV, CBG CBC, CBN, CBDB, CBE, CBL, and CBND.

Specifically, the amphiphilic high molecular polymer is selected from one of or a combination of two or more of a poloxamer, a polyethylene glycol-poly(lactic-co-glycolic acid) copolymer, a polyethylene glycol-polycaprolactone block copolymer, a polyethylene glycol-polylactic acid block copolymer, and a polyethylene glycol-polystyrene block copolymer; preferably, the poloxamer is selected from one of or a combination of two or more of poloxamers 188, 338, 407, 124, 215, and 237; preferably, a number average molecular weight of the polyethylene glycol-poly(lactic-co-glycolic acid) block copolymer, the polyethylene glycol-polycaprolactone block copolymer, the polyethylene glycol-polylactic acid block copolymer, and the polyethylene glycol-polystyrene block copolymer is 500-50,000, wherein the polyethylene glycol is polyethylene glycol monomethyl ether, and a number average molecular weight of the polyethylene glycol moiety is 200-12,000.

Specifically, the cyclodextrin is selected from α-cyclodextrin, β-cyclodextrin, γ-cyclodextrin, α-cyclodextrin derivatives, β-cyclodextrin derivatives, and γ-cyclodextrin derivatives.

Specifically, the cyclodextrin is selected from β-cyclodextrin, hydroxypropyl-β-cyclodextrin, methyl-β-cyclodextrin, and propylene oxide-β-cyclodextrin.

The present invention also provides a water-soluble cannabinoid formulation and a preparation method therefor.

Specifically, the water-soluble cannabinoid formulation described above comprises a cannabinoid, an amphiphilic high molecular polymer, and cyclodextrin, and optionally, a pharmaceutically acceptable lyoprotectant and a pH regulator, and it is prepared by a method comprising the following steps:
(1) adding a cannabinoid, an amphiphilic high molecular polymer, and cyclodextrin to water to prepare a water-soluble complex solution, wherein a mass ratio of materials to water is 1:(1-6);
(2) drying the water-soluble complex solution obtained in step (1) to obtain water-soluble cannabinoid solid granules; and
(3) preparing the cannabinoid solid granules obtained in step (2) into a water-soluble cannabinoid formulation.

Specifically, in the water-soluble cannabinoid formulation described above, a content of the cannabinoid in percentages by weight is 1-40% (specifically, such as 1%, 5%, 10%, 15%, 20%, 25%, 30%, 35%, or 40%).

Specifically, in the water-soluble cannabinoid formulation described above, a content of the amphiphilic high molecular polymer in percentages by weight is 1-90% (specifically, such as 1%, 5%, 10%, 15%, 20%, 25%, 30%, 35%, 40%, 45%, 50%, 55%, 60%, 65%, 70%, 75%, 80%, 85%, or 90%).

Specifically, in the water-soluble cannabinoid formulation described above, a content of the cyclodextrin in percentages by weight is 1-90% (specifically, such as 1%, 5%, 10%, 15%, 20%, 25%, 30%, 35%, 40%, 45%, 50%, 55%, 60%, 65%, 70%, 75%, 80%, 85%, or 90%).

In one embodiment of the present invention, the cannabinoid described above is selected from one of or a combination of two or more of pure products of CBD, CBDV, CBG, CBC, CBN, CBDB, CBE, CBL, and CBND. It can be a plant extract cannabinoid or a synthetic cannabinoid and is preferably a plant extract cannabinoid.

In another embodiment of the present invention, the cannabinoid described above is a cannabis extract comprising one of or a combination of two or more of CBD, CBDV, CBG CBC, CBN, CBDB, CBE, CBL, and CBND.

In one embodiment of the present invention, the amphiphilic high molecular polymer described above is an amphiphilic high molecular polymer block copolymer. It is a block copolymer consisting of two or more polymers whose hydrophilic moiety can be polyethylene glycol, polyoxyethylene, povidone, and the like, and whose hydrophobic moiety can be polyoxypropylene, polylactic acid, polystyrene, polycaprolactone, polyamino acid, poly(lactic-co-glycolic acid), polyacrylic acid, and the like, for example, one of or a combination of two or more of a poloxamer (PEO-PPO-PEO), a polylactic acid-polyethylene glycol-polylactic acid triblock copolymer (PLA-PEG-PLA), a polyethylene glycol-polyacrylic acid block copolymer (PEG-PAA), a polyethylene glycol-polyaspartic acid block copolymer (PEG-PASP), a polyethylene glycol-poly(lactic-co-glycolic acid) block copolymer (PEG-PLAG), a polyethylene glycol-polycaprolactone block copolymer (PEG-PCL), a polyethylene glycol-polylactic acid block copolymer (PEG-PLA/PTX), a polyethylene glycol-polystyrene block copolymer (PEG-b-PS), and the like.

Specifically, the poloxamer described above is selected from one of or a combination of two or more of poloxamers 188, 338, 407, 124, 215, 237, and the like.

Specifically, a number average molecular weight of the polyethylene glycol-poly(lactic-co-glycolic acid) block copolymer, the polyethylene glycol-polycaprolactone block copolymer, the polyethylene glycol-polylactic acid block copolymer, and the polyethylene glycol-polystyrene block copolymer described above is 500-50,000 (specifically, such as 500, 1000, 2000, 3000, 4000, 5000, 6000, 8000, 10,000, 12,000, 14,000, 16,000, 18,000, 20,000, 22,000, 24,000, 26,000, 28,000, 30,000, 32,000, 34,000, 36,000, 38,000, 40,000, 42,000, 44,000, 46,000, 48,000, or 50,000), wherein the polyethylene glycol is polyethylene glycol monomethyl ether, and a number average molecular weight of the polyethylene glycol moiety can be 200-12,000 (specifically, such as 200, 400, 500, 600, 750, 1000, 2000, 4000, 5000, 6000, 8000, 10,000, or 12,000).

In one embodiment of the present invention, the cyclodextrin described above includes cyclodextrin or a derivative thereof.

Specifically, the cyclodextrin described above is selected from α-cyclodextrin, β-cyclodextrin, γ-cyclodextrin, α-cyclodextrin derivatives, β-cyclodextrin derivatives, and γ-cyclodextrin derivatives.

Specifically, the cyclodextrin described above is selected from one of or a combination of two or more of β-cyclodextrin, hydroxypropyl-β-cyclodextrin, methyl-β-cyclodextrin, and propylene oxide-β-cyclodextrin.

In one embodiment of the present invention, the water-soluble cannabinoid formulation described above also comprises a pharmaceutically acceptable lyoprotectant.

Specifically, in the water-soluble cannabinoid formulation described above, a content of the lyoprotectant in percentages by weight is 0-20% (specifically, such as 1%, 2%, 3%, 4%, 5%, 6%, 7%, 8%, 9%, 10%, 15%, or 20%).

Specifically, the lyoprotectant described above is selected from one or more of sodium thiosulfate, sodium bisulfite, sodium sulfite, sodium metabisulfite, vitamin C, lactose, mannitol, sorbitol, EDTA-2Na, trehalose, glucose, xylitol, and maltose.

In one embodiment of the present invention, the water-soluble cannabinoid formulation described above also comprises a pH regulator.

Specifically, in the water-soluble cannabinoid formulation described above, a content of the pH regulator in percentages by weight is 0-40% (specifically, such as 0.01%, 0.05%, 0.1%, 0.2%, 0.3%, 0.4%, 0.5%, 1%, 2%, 3%, 4%, 5%, 10%, 15%, 20%, 25%, 30%, 35%, or 40%).

Specifically, the pH regulator described above is selected from one or more of citric acid, sodium citrate, tartaric acid, sodium tartrate, acetic acid, sodium acetate, sodium dihydrogen phosphate, disodium hydrogen phosphate, hydrochloric acid, lactic acid, and sodium hydroxide.

In one embodiment of the present invention, the step (1) described above comprises the following steps:
(1-1) adding an amphiphilic high molecular polymer, cyclodextrin, a lyoprotectant, and a pH regulator to a solvent for dissolving; and
(1-2) dissolving a single component or an extract of cannabinoid into the mixed solution obtained in step (1-1) to obtain a clear and transparent water-soluble complex solution.

In one embodiment of the present invention, the solvent in step (1-1) is water, such as water for injection.

Specifically, the amount of the solvent used in step (1-1) is 1-6 times (mass ratio, specifically, such as 1, 2, 3, 4, 5, or 6 times) the amount of the material components.

Specifically, the time for stirring in step (1-2) is 1-24 h (specifically, such as 1, 2, 4, 6, 8, 10, 12, 14, 16, 18, 20, 22, or 24 h).

Specifically, in step (1-2), the temperature for stirring is 15-85 °C (specifically, such as 15, 20, 25, 30, 35, 40, 45, 50, 55, 60, 65, 70, 75, 80, or 85 °C).

Specifically, the particle size of the water-soluble complex solution described above is 1-500 nm (specifically, such as 1, 10, 50, 100, 150, 200, 250, 300, 350, 400, 450, or 500 nm).

Specifically, the drying in step (2) is lyophilizing or reduced-pressure drying.

Specifically, the lyophilizing described above comprises steps of pre-freezing, sublimation drying, and desorption drying, wherein the temperature for pre-freezing is -30 to -50 °C (specifically, such as -30, -35, -40, -45, or -50 °C), the time for pre-freezing is 0.5-3 h (specifically, such as 0.5, 1, 2, or 3 h), and the vacuum degree in the pre-freezing step is controlled at 1-100 Pa (1, 10, 20, 30, 40, 50, 60, 80, or 100 Pa); the temperature for sublimation drying is -20 to 10 °C (specifically, such as -20, -10, -5, 0, 5, or 10 °C), and the time for sublimation drying is 1-36 h (specifically, such as 1, 6, 12, 18, 24, 30, or 36 h); the temperature for desorption drying is 10-30 °C (specifically, such as 10, 15, 20, 25, or 30 °C), and the time for desorption drying is 1-24 h (specifically, such as 1, 6, 12, 18, or 24 h).

Specifically, the reduced-pressure drying described above comprises steps of reducing pressure and drying, wherein the vacuum degree in the reducing pressure step is no more than 0.08 MPa (specifically, such as 0.01, 0.02, 0.03, 0.04, 0.05, 0.06, 0.07, or 0.08 MPa), the temperature for drying in the drying step is 10-50 °C (specifically, such as 10, 15, 20, 25, 30, 35, 40, 45, or 50 °C), and the time for drying is 1-48 h (specifically, such as 1, 6, 12, 18, 24, 30, 36, 42, or 48 h). Specifically, the water-soluble cannabinoid formulation described above is a solid formulation or a semi-solid formulation.

In one embodiment of the present invention, the water-soluble cannabinoid formulation described above is an oral formulation, such as an effervescent tablet, a sustained-release tablet, a disintegrating tablet, and a dripping pill.

In another embodiment of the present invention, the water-soluble cannabinoid formulation described above is an inhalant formulation, such as an inhalant powder.

In another embodiment of the present invention, the water-soluble cannabinoid formulation described above is an enteric formulation, such as an enteric capsule.

In one embodiment of the present invention, the water-soluble cannabinoid formulation described above is an inhalant having a particle size of 1-2 µm (specifically, such as 1, 1.5, or 2 µm), wherein the drying in the step (2) described above is lyophilizing or reduced-pressure drying.

In another embodiment of the present invention, the water-soluble cannabinoid formulation described above is an effervescent tablet or a disintegrating tablet, wherein the drying in the step (2) described above is lyophilizing or reduced-pressure drying.

In another embodiment of the present invention, the water-soluble cannabinoid formulation described above is an enteric capsule, wherein the drying in the step (2) described above is lyophilizing or reduced-pressure drying.

The method for preparing a water-soluble cannabinoid formulation provided by the present invention comprises the following steps:
(1) adding a cannabinoid, an amphiphilic high molecular polymer, and cyclodextrin to water to prepare a water-soluble complex solution, wherein a mass ratio of materials to water is 1:(1-6);
(2) drying the water-soluble complex solution obtained in step (1) to obtain water-soluble cannabinoid solid granules; and
(3) preparing the cannabinoid solid granules obtained in step (2) into a water-soluble cannabinoid formulation.

In one embodiment of the present invention, the step (1) described above comprises the following steps:
(1-1) adding an amphiphilic high molecular polymer, cyclodextrin, a lyoprotectant, and a pH regulator to a solvent for dissolving; and
(1-2) dissolving a single component or an extract of cannabinoid into the mixed solution obtained in step (1-1) to obtain a clear and transparent water-soluble complex solution.

In one embodiment of the present invention, the solvent in step (1-1) is water, such as water for injection.

Specifically, the amount of the solvent used in step (1-1) is 1-6 times (mass ratio, specifically, such as 1, 2, 3, 4, 5, or 6 times) the amount of the material components.

Specifically, the time for stirring in step (1-2) is 1-24 h (specifically, such as 1, 2, 4, 6, 8, 10, 12, 14, 16, 18, 20, 22, or 24 h).

Specifically, in step (1-2), the temperature for stirring is 15-85 °C (specifically, such as 15, 20, 25, 30, 35, 40, 45, 50, 55, 60, 65, 70, 75, 80, or 85 °C).

Specifically, the particle size of the water-soluble complex solution described above is 1-500 nm (specifically, such as 1, 10, 50, 100, 150, 200, 250, 300, 350, 400, 450, or 500 nm).

Specifically, the drying in step (2) is lyophilizing or reduced-pressure drying.

Specifically, the lyophilizing described above comprises steps of pre-freezing, sublimation drying, and desorption drying, wherein the temperature for pre-freezing is -30 to -50 °C (specifically, such as -30, -35, -40, -45, or -50 °C), the time for pre-freezing is 0.5-3 h (specifically, such as 0.5, 1, 2, or 3 h), and the vacuum degree in the pre-freezing step is controlled at 1-100 Pa (1, 10, 20, 30, 40, 50, 60, 80, or 100 Pa); the temperature for sublimation drying is -20 to 10 °C (specifically, such as -20, -10, -5, 0, 5, or 10 °C), and the time for sublimation drying is 1-36 h (specifically, such as 1, 6, 12, 18, 24, 30, or 36 h); the temperature for desorption drying is 10-30 °C (specifically, such as 10, 15, 20, 25, or 30 °C), and the time for desorption drying is 1-24 h (specifically, such as 1, 6, 12, 18, or 24 h).

Specifically, the reduced-pressure drying described above comprises steps of reducing pressure and drying, wherein the vacuum degree in the reducing pressure step is no more than 0.08 MPa (specifically, such as 0.01, 0.02, 0.03, 0.04, 0.05, 0.06, 0.07, or 0.08 MPa), the temperature for drying in the drying step is 10-50 °C (specifically, such as 10, 15, 20, 25, 30, 35, 40, 45, or 50 °C), and the time for drying is 1-48 h (specifically, such as 1, 6, 12, 18, 24, 30, 36, 42, or 48 h).

In one embodiment of the present invention, a method for preparing the water-soluble complex solution comprises adding one or more of β-cyclodextrin (a content in percentages by weight is 0-3%), hydroxypropyl-β-cyclodextrin (a content in percentages by weight is 1-40%), methyl-β-cyclodextrin (a content in percentages by weight is 1-40%), propylene oxide-β-cyclodextrin (a content in percentages by weight is 1-40%), poloxamer (a content in percentages by weight is 1-40%), PEG-PLAG (a content in percentages by weight is 1-3%), PEG-PCL (a content in percentages by weight is 1-2%), PEG-PLA/PTX (a content in percentages by weight is 1-2%), PEG-b-PS (a content in percentages by weight is 1-2%), EDTA-2Na (a content in percentages by weight is 0-1%), sodium thiosulfate (a content in percentages by weight is 0-1%), mannitol (a content in percentages by weight is 0-1%), and citric acid (a content in percentages by weight is 0-2%) to 1-6 times of water, and dissolving;
adding a single component or an extract of cannabinoid (a content in percentages by weight is 1-20%) to the mixed solution, and stirring at 45-85 °C for 0-2 h to obtain a clear and transparent water-soluble complex solution with a particle size of less than 500 nm.

In one embodiment of the present invention, a method for preparing the water-soluble complex solution comprises adding one or more of β-cyclodextrin (a content in percentages by weight is 0-3%), hydroxypropyl-β-cyclodextrin (a content in percentages by weight is 1-40%), methyl-β-cyclodextrin (a content in percentages by weight is 1-40%), propylene oxide-β-cyclodextrin (a content in percentages by weight is 1-40%), poloxamer (a content in percentages by weight is 1-40%), PEG-PLAG (a content in percentages by weight is 1-3%), PEG-PCL (a content in percentages by weight is 1-2%), PEG-PLA/PTX (a content in percentages by weight is 1-2%), PEG-b-PS (a content in percentages by weight is 1-2%), EDTA-2Na (a content in percentages by weight is 0-1%), sodium thiosulfate (a content in percentages by weight is 0-1%), mannitol (a content in percentages by weight is 0-1%), and citric acid (a content in percentages by weight is 0-2%) to 1-6 times of water, and dissolving;
adding CBD (a content in percentages by weight is 1-20%) to the mixed solution, and stirring at 45-85 °C for 0-2 h to obtain a clear and transparent water-soluble complex solution with a particle size of less than 500 nm.

In one embodiment of the present invention, a method for preparing the water-soluble complex solution comprises adding one or more of β-cyclodextrin (a content in percentages by weight is 0-3%), hydroxypropyl-β-cyclodextrin (a content in percentages by weight is 1-40%), methyl-β-cyclodextrin (a content in percentages by weight is 1-40%), propylene oxide-β-cyclodextrin (a content in percentages by weight is 1-40%), poloxamer (a content in percentages by weight is 1-40%), PEG-PLAG (a content in percentages by weight is 1-3%), PEG-PCL (a content in percentages by weight is 1-2%), PEG-PLA/PTX (a content in percentages by weight is 1-2%), PEG-b-PS (a content in percentages by weight is 1-2%), EDTA-2Na (a content in percentages by weight is 0-1%), sodium thiosulfate (a content in percentages by weight is 0-1%), mannitol (a content in percentages by weight is 0-1%), and citric acid (a content in percentages by weight is 0-2%) to 1-6 times of water, and dissolving;
adding a solution of a single component or an extract of cannabinoid in ethanol (a content in percentages by weight is 1-20%) to the mixed solution, and stirring at room temperature for 1-24 h to obtain a clear and transparent water-soluble complex solution with a particle size of less than 500 nm.

In one embodiment of the present invention, a method for preparing the water-soluble complex solution comprises adding one or more of β-cyclodextrin (a content in percentages by weight is 0-3%), hydroxypropyl-β-cyclodextrin (a content in percentages by weight is 1-40%), methyl-β-cyclodextrin (a content in percentages by weight is 1-40%), propylene oxide-β-cyclodextrin (a content in percentages by weight is 1-40%), poloxamer (a content in percentages by weight is 1-40%), PEG-PLAG (a content in percentages by weight is 1-3%), PEG-PCL (a content in percentages by weight is 1-2%), PEG-PLA/PTX (a content in percentages by weight is 1-2%), PEG-b-PS (a content in percentages by weight is 1-2%), EDTA-2Na (a content in percentages by weight is 0-1%), sodium thiosulfate (a content in percentages by weight is 0-1%), mannitol (a content in percentages by weight is 0-1%), and citric acid (a content in percentages by weight is 0-2%) to 1-6 times of water, and dissolving;
adding a solution of CBD in ethanol (a content in percentages by weight is 1-20%) to the mixed solution, and stirring at room temperature for 1-24 h to obtain a clear and transparent water-soluble complex solution with a particle size of less than 500 nm.

In one embodiment of the present invention, a method for preparing cannabinoid solid granules comprises placing the water-soluble complex solution into a lyophilization oven, freezing at -40 °C for 0.5-3 h with the vacuum degree controlled at 1-100 Pa, performing primary drying at -20 to 10 °C for 1-36 h, performing secondary drying at 10-30 °C for 1-24 h, and sieving the dried materials with Nos. 1-5 sieves for sizing.

In one embodiment of the present invention, a method for preparing cannabinoid solid granules comprises placing the water-soluble complex solution into a reduced-pressure drying oven, drying at 20-50 °C for 1-48 h under the vacuum degree of ≤ 0.08 MPa, and sieving the dried materials with Nos. 1-5 sieves for sizing.

In one embodiment of the present invention, a method for preparing cannabinoid inhalant powders comprises milling the cannabinoid solid granules using ball-milling to no more than 10 µm directly to prepare cannabinoid atomization inhalant powders, and filling No. 2 to No. 00 capsules, vesicles, and storage tanks with the cannabinoid atomization inhalant powders for direct administration by powder inhalation.

In one embodiment of the present invention, a method for preparing cannabinoid orally disintegrating tablets comprises mixing well the cannabinoid atomization inhalant powders (a content in percentages by weight is 1-90%), low-substituted hydroxypropylcellulose (a content in percentages by weight is 1-80%), mannitol (a content in percentages by weight is 0-80%), microcrystalline cellulose (a content in percentages by weight is 0-80%), pregelatinized starch (a content in percentages by weight is 0-80%), colloidal silicon dioxide (a content in percentages by weight is 0-10%), and magnesium stearate (a content in percentages by weight is 0-10%), and directly tableting into disintegrating tablets each weighing 0.5 g.

In one embodiment of the present invention, a method for preparing cannabinoid orally disintegrating tablets comprises granulating the cannabinoid atomization inhalant powders (a content in percentages by weight is 1-90%), low-substituted hydroxypropylcellulose (a content in percentages by weight is 1-80%), mannitol (a content in percentages by weight is 0-80%), microcrystalline cellulose (a content in percentages by weight is 0-80%), pregelatinized starch (a content in percentages by weight is 0-80%), colloidal silicon dioxide (a content in percentages by weight is 0-10%), and magnesium stearate (a content in percentages by weight is 0-10%) by a wet method or one-step granulating, and tableting into disintegrating tablets each weighing 0.5 g.

In one embodiment of the present invention, a method for preparing cannabinoid effervescent tablets comprises preparing granules 1 by granulating DL tartaric acid (a content in percentages by weight is 0-20%), citric acid (a content in percentages by weight is 0-35%), mannitol (a content in percentages by weight is 0-3%), lactose (a content in percentages by weight is 0-1%), sodium chloride (a content in percentages by weight is 0-2%), sucrose (a content in percentages by weight is 0-1%), and polyethylene glycol (ta content in percentages by weight is 0-15%) with a fluidized bed or by a wet method; preparing granules 2 by granulating sodium bicarbonate (a content in percentages by weight is 0-1%), aspartame (a content in percentages by weight is 0-1%), lactose (a content in percentages by weight is 0-1%), xylitol (a content in percentages by weight is 0-3%), essence (a content in percentages by weight is 0-3%), sodium carboxymethyl cellulose (a content in percentages by weight is 0-1%), and polyethylene glycol (a content in percentages by weight is 0-15%) with a fluidized bed or by a wet method, and mixing well the granules 1, the granules 2, the cannabinoid solid granules (a content in percentages by weight is 1-15%), leucine (a content in percentages by weight is 0-1%), essence (a content in percentages by weight is 0-2%), carotene (a content in percentages by weight is 0-2%), and vitamin C (a content in percentages by weight is 0-10%) and tableting into effervescent tablets each weighing 0.1-10 g.

In one embodiment of the present invention, a method for preparing cannabinoid effervescent tablets comprises mixing well DL tartaric acid (a content in percentages by weight is 0-20%), citric acid (a content in percentages by weight is 0-35%), mannitol (a content in percentages by weight is 0-3%), lactose (a content in percentages by weight is 0-1%), sodium chloride (a content in percentages by weight is 0-2%), sucrose (a content in percentages by weight is 0-1%), sodium bicarbonate (a content in percentages by weight is 0-1%), aspartame (a content in percentages by weight is 0-1%), xylitol (a content in percentages by weight is 0-3%), essence (a content in percentages by weight is 0-3%), polyethylene glycol (a content in percentages by weight is 0-15%), the cannabinoid solid granules (a content in percentages by weight is 1-15%), leucine (a content in percentages by weight is 0-1%), carotene (a content in percentages by weight is 0-2%), and vitamin C (a content in percentages by weight is 0-10%) and tableting into effervescent tablets each weighing 0.1-10 g.

In one embodiment of the present invention, a method for preparing cannabinoid enteric capsules comprises: placing blank pellet cores in a coating pan; dissolving the cannabinoid solid granules (a content in percentages by weight is 1-20%), hydroxypropyl methylcellulose (a content in percentages by weight is 1-25%), and crospolyvinylpyrrolidone (a content in percentages by weight is 1-25%), spraying the solution on the blank pellet cores, and drying at 35-55 °C for 0-12 h; taking hydroxypropyl methylcellulose (a content in percentages by weight is 0-25%) and mannitol (a content in percentages by weight is 0-12%), adding water, stirring well, spraying the solution on the drug-containing pellet cores, and drying at 35-55 °C for 0-12 h; taking hydroxypropyl methylcellulose (a content in percentages by weight is 0-25%), diacetyl monoglyceride (a content in percentages by weight is 0-5%), and talc (a content in percentages by weight is 0-1%), adding water, stirring well, spraying the solution on the drug-containing pellet cores, and drying at 35-55 °C for 0-12 h; taking acrylic resin (a content in percentages by weight is 0-15%), adding water, stirring well, spraying the solution on the drug-containing pellet cores, and drying at 35-55 °C for 0-12 h; and adding talc (a content in percentages by weight is 0-1%), and filling capsules.

The present invention also provides a method for preventing and/or treating diseases, which comprises administering to a subject in need thereof an effective amount of the water-soluble cannabinoid formulation of the present invention described above.

Specifically, in the method described above, the water-soluble cannabinoid formulation has the definition described above in the present invention.

Specifically, in the method described above, the diseases are indications of the corresponding cannabinoid in the water-soluble cannabinoid formulation described above, such as pain (e.g., chronic pain), inflammation (e.g., dermatitis), tumors (e.g., neuroglioma, leukemia, prostate cancer, etc.), liver damage (e.g., ischemic liver damage and liver damage caused by chronic alcoholism), and nervous system diseases (e.g., epilepsy, multiple sclerosis, Parkinson's disease, Alzheimer's disease, etc.) (see, e.g., Guo Rong, Chen Xuan, Guo Hongyan, Review on Pharmacological Effects of Tetrahydrocannabinol and Cannabidiol, Natural Product Research and Development, 2017, 29: 449-1453).

Specifically, the subject described above is a mammal, particularly a human.

Specifically, the effective amount of the water-soluble cannabinoid formulation described above depends on various factors, including the age, weight, sex, natural health, and nutritional status of the patient, administration time and metabolic rate of drug, severity of the disease, the subjective judgment of the treating physician, etc.

The present invention also provides a healthcare product comprising the water-soluble cannabinoid formulation of the present invention described above.

The present invention also provides a method for enhancing immunity and resisting oxidation, which comprises administering to a subject in need thereof an effective amount of the healthcare product described above.

The water-soluble cannabinoid formulation provided by the present invention has the advantages of high encapsulation rate and transfer rate of the effective ingredient, high drug loading, and strong stability. A new self-assembly technology at room temperature is adopted to prevent the active ingredient cannabinoid from degrading and changing color at high temperature; the bioavailability of the active ingredient is high, which can reduce the dose for a single administration. Particularly, the inhalant powders feature high drug loading (10% or more), strong stability (shelf life: 2-3 years), and high *in vitro* deposition rate (the emptying rate can reach 95%, and the effective deposition rate can reach 45%), produce a quick response after inhalation, and can provide sustained and stable plasma concentration.

### DETAILED DESCRIPTION

Unless otherwise defined, all scientific and technical terms used herein have the same meaning as commonly understood by those skilled in the art to which the present invention relates. For example, the following abbreviations and their corresponding substances appearing in the present invention are as follows:
- CBDV: cannabidivarin
- CBD: cannabidiol
- CBG: cannabigerol
- CBN: cannabinol
- CBC: cannabichromene
- CBDB: 4-butyl-5'-methyl-2'-(prop-1-en-2-yl)-1',2', 3',4'-tetrahydro-(1,1'-biphenyl)-2,6-diol, cannabidibutol
- CBE: cannabielsoin
- CBL: cannabicyclol
- CBND: cannabinodiol
- TPGS: vitamin E polyethylene glycol succinate
- Poloxamer: poloxamer
- PEG-PAA: polyethylene glycol-polyacrylic acid block copolymer
- PEG-PASP: polyethylene glycol-polyaspartic acid block copolymer
- PEG-PLAG: polyethylene glycol-poly(lactic-co-glycolic acid) block copolymer
- PEG-PCL: polyethylene glycol-polycaprolactone block copolymer
- PEG-PLA/PTX: polyethylene glycol-polylactic acid block copolymer
- PEG-b-PS: polyethylene glycol-polystyrene block copolymer
- SFD: spray freeze drying
- CD: cyclodextrin
- β-CD: β-cyclodextrin
- HP-β-CD: hydroxypropyl-β-cyclodextrin
- Methyl-β-CD: methyl-β-cyclodextrin
- Propylene oxide-β-CD: propylene oxide-β-cyclodextrin
- EDTA-2Na: ethylenediaminetetraacetic acid disodium salt

In the present invention, the term "cannabinoid" refers to a class of secondary metabolites containing alkyl and monoterpene group molecular structures that are unique to cannabis plants, such as CBD, CBDV, CBG CBC, CBN, CBDB, CBE, CBL, and CBND (as described in Chen Xuan, Yang Ming, Guo Hongyan, Research Advances in Cannabinoids of Cannabis Sativa, Bulletin of Botany, 2011, 46 (2): 197-205).

In the present invention, pure products of CBD, CBDV, CBG CBC, CBN, CBDB, CBE, CBL, and CBND refer to pure substances of the compounds described above, particularly corresponding commercially available products, wherein the purity of the compounds is at least 99.5% or more, particularly 99.9% or more (the rest are impurities).

In the present invention, the cannabis is industrial cannabis, which is cannabis with a content of tetrahydrocannabinol of less than 0.3%. In the present invention, legal permission has been obtained for the acquisition, processing, and research of industrial cannabis.

The technical schemes of the present invention will be clearly and completely described below with reference to the examples of the present invention, and it is obvious that the described examples are only a part of the examples of the present invention but not all of them. Based on the examples of the present invention, all other examples obtained by those of ordinary skills in the art without creative work shall fall within the protection scope of the present invention.

### Example 1: Preparation of Water-Soluble Complex Solution

### 1. The specific formula of the water-soluble complex solution is shown in Table 1:

**Table 1. Formulas of water-soluble complex solution**

| Formula | Cannabidiol (CBD) | β-cyclodextrin% | Hydroxypropyl-β-cyclodextrin% | Methyl-β-cyclodextrin% | Propylene oxide-β-cyclodextrin% | Poloxam% | PEG-PLAG% |
|---|---|---|---|---|---|---|---|
| 1 | 10 | 0 | 40 | 5 | 10 | 26 | 3 |
| 2 | 20 | 3 | 15 | 5 | 10 | 35 | 3 |
| 3 | 10 | 0 | 10 | 40 | 5 | 26 | 3 |
| 4 | 20 | 3 | 10 | 15 | 5 | 35 | 3 |
| 5 | 10 | 0 | 5 | 10 | 40 | 26 | 3 |
| 6 | 20 | 3 | 5 | 10 | 15 | 35 | 3 |

| Formula | PEG-PCL% | PEG-PLA/PTX% | PEG-b-PS% | EDTA-2Na% | Sodium thiosulfate% | Mannitol% | Citric acid% |
|---|---|---|---|---|---|---|---|
| 1 | 1 | 1 | 1 | 1 | 0.5 | 1 | 0.5 |
| 2 | 2 | 2 | 2 | 1 | 0.5 | 1 | 0.5 |
| 3 | 1 | 1 | 1 | 1 | 0.5 | 1 | 0.5 |
| 4 | 2 | 2 | 2 | 1 | 0.5 | 1 | 0.5 |
| 5 | 1 | 1 | 1 | 1 | 0.5 | 1 | 0.5 |
| 6 | 2 | 2 | 2 | 1 | 0.5 | 1 | 0.5 |

### 2.Preparation process

### (1) First set of preparation process (process 1)

The water-soluble complex solutions were prepared by feeding materials according to the proportion specified in the formulas 1-6, specifically as follows:
a. Dissolving: adding one or more of β-cyclodextrin, hydroxypropyl-β-cyclodextrin, methyl-β-cyclodextrin, propylene oxide-β-cyclodextrin, poloxamer, PEG-PLAG, PEG-PCL, PEG-PLA/PTX, PEG-b-PS, EDTA-2Na, sodium thiosulfate, mannitol, and citric acid to 3 times of water, and dissolving;
b. Mixing and dissolving: adding cannabidiol (CBD) and stirring at 85 °C for 2 h to obtain a clear and transparent water-soluble complex solution with a particle size of less than 500 nm.

The quality comparison of the water-soluble complex solutions obtained by the preparation process described above is shown in Table 2:

**Table 2. Quality comparison of water-soluble complex solutions**

| Formula | Quality of corresponding solution | | | |
|---|---|---|---|---|
| | Clarity of solution | Particle size of solution | Inclusion rate of the effective ingredient% | Transfer rate of the effective ingredient% |
| 1 | Clear and transparent | 0-500nm | 100 | 100 |
| 2 | Clear and transparent | 0-500nm | 99 | 99 |
| 3 | Clear and transparent | 0-500nm | 100 | 99 |
| 4 | Clear and transparent | 0-500nm | 100 | 100 |
| 5 | Clear and transparent | 0-500nm | 99 | 100 |
| 6 | Clear and transparent | 0-500nm | 99 | 100 |

| | | | | |
|---|---|---|---|---|
| (Note: the inclusion rate of the effective ingredient refers to the percentage of CBD detected in purified water to CBD detected in methanol; the transfer rate of the effective ingredient refers to the percentage of CBD actually detected to the amount added.) | | | | |

### (2) Second set of preparation process (process 2)

The water-soluble complex solutions were prepared by feeding materials according to the proportion specified in the formulas 1-6, specifically as follows:
a. Dissolving: adding one or more of β-cyclodextrin, hydroxypropyl-β-cyclodextrin, methyl-β-cyclodextrin, propylene oxide-β-cyclodextrin, poloxamer, PEG-PLAG, PEG-PCL, PEG-PLA/PTX, PEG-b-PS, EDTA-2Na, sodium thiosulfate, mannitol, and citric acid to 3 times of water, and dissolving;
b. Mixing and dissolving: adding a solution of cannabidiol (CBD) in ethanol and stirring at room temperature for 24 h to obtain a clear and transparent water-soluble complex solution with a particle size of less than 500 nm.

The quality comparison of the water-soluble complex solutions obtained by the preparation process described above is shown in Table 3:

**Table 3. Quality comparison of water-soluble complex solutions**

| Formula | Quality of corresponding solution | | | |
|---|---|---|---|---|
| | Clarity of solution | Particle size of solution | Inclusion rate of the effective ingredient% | Transfer rate of the effective ingredient% |
| 1 | Clear and transparent | 0-500nm | 100 | 99 |
| 2 | Clear and transparent | 0-500nm | 99 | 99 |
| 3 | Clear and transparent | 0-500nm | 100 | 99 |
| 4 | Clear and transparent | 0-500nm | 100 | 100 |
| 5 | Clear and transparent | 0-500nm | 99 | 100 |
| 6 | Clear and transparent | 0-500nm | 99 | 99 |

It can be seen from results in Tables 2 and 3 that for the same formula, the inclusion rate and transfer rate of the effective ingredient of the first set of process and the second set of process are substantially the same, but the inclusion time can be shortened under heating.

### Example 2: Preparation of Cannabinoid Solid Granules

The water-soluble complex solutions obtained in Example 1 were each dried to obtain water-soluble cannabinoid solid granules, wherein the drying is lyophilizing or reduced-pressure drying.
1. Lyophilizing process: placing the water-soluble complex solution in Example 1 into a lyophilization oven, freezing at -40 °C for 3 h under the vacuum degree of 100 Pa, performing primary drying at 10 °C for 36 h, performing secondary drying at 30 °C for 24 h, and sieving the dried materials with No. 5 sieve for sizing;
2. Reduced-pressure drying process: placing the water-soluble complex solution in Example 1 into a reduced-pressure drying oven, drying at 50 °C for 48 h under the vacuum degree of 0.04 MPa, and sieving the dried materials with No. 5 sieve for sizing.

The comparison of the lyophilizing process and the reduced-pressure drying process is shown in Table 4:

**Table 4. Comparison of lyophilizing process and reduced-pressure drying process**

| Drying process | Appearance | Degree of process controllability | Solubility |
|---|---|---|---|
| Lyophilizing | Off-white granules | Simple and controllable | Clear solution |
| Reduced-pressure drying | Off-white granules | Simple and controllable | Clear solution |

It can be seen from results in Table 4 that the granules prepared by the lyophilizing process and the reduced-pressure drying process are the same in appearance and solubility, and the reduced-pressure drying process features low cost.

### Example 3: Preparation of Cannabinoid Inhalant Powders

### 1. Micronization:

Milling the water-soluble cannabinoid solid granules prepared by the reduced-pressure drying method in Example 2 using ball-milling to no more than 10 µm directly to prepare atomization inhalant powders;

### 2. Filling:

Filling No. 2 to No. 00 capsules, vesicles, and storage tanks with the atomization inhalant powders for direct administration by powder inhalation.

The quality study of cannabinoid inhalant powders is shown in Table 5:

**Table 5. Quality study of cannabinoid inhalant powders**

| Mode of milling | Degree of process controllability | Powder hygroscopicity% | Solubility | Surface form | Particle size distribution d(0.9) µm | Determination of *in vitro* deposition | |
|---|---|---|---|---|---|---|---|
| | | | | | | Emptying rate% | Deposition rate% |
| Ball-milling | Simple and controllable | 1.0 | Clear solution | Spherical | 2.0µm | 95 | 40 |

The cannabinoid solid granules are directly micronized to prepare inhalant powders. The process is simple and controllable and features low cost. The cannabinoid inhalant powders prepared can be used for diseases such as epilepsy, depression, and chronic pain. They produce a quick response and have no hepatic first pass effect, gastrointestinal irritation, or degradation.

### Example 4: Preparation of Cannabinoid Effervescent Tablets

1. Formula: the cannabinoid solid granules (prepared in Example 2), DL tartaric acid, citric acid, sodium bicarbonate, mannitol, lactose, aspartame, xylitol, polyethylene glycol, leucine, essence, sodium chloride, and sucrose.

**Table 6. Formulas of cannabinoid effervescent tablets**

| Formula | Cannabinoid solid granules% | DL tartaric acid% | Citric acid% | Sodium bicarbonate% | Mannitol% | Lactose% | Aspartame% | Xylitol% | polyethylene glycol% |
|---|---|---|---|---|---|---|---|---|---|
| 1 | 15 | 20 | 35 | 0.2 | 3 | 0.3 | 0.5 | 3 | 15 |
| 2 | 15 | 20 | 33 | 0.2 | 3 | 0.3 | 0.5 | 2 | 15 |
| 3 | 14 | 20 | 30 | 0.2 | 2 | 0.3 | 0.5 | 2 | 14 |

| Formula | Leucine% | Essence% | Sodium chloride% | Sucrose% | Carotene% | Vitamin C% | | | |
|---|---|---|---|---|---|---|---|---|---|
| 1 | 1 | 2 | 2 | 1 | 2 | 10 | | | |
| 2 | 1 | 1 | 1 | 1 | 2 | 10 | | | |
| 3 | 1 | 1 | 1 | 1 | 2 | 10 | | | |

### Specification: tablet weight is 1 g

### 2. Preparation process

### (1) First set of preparation process (process 1)

Preparing granules 1 by granulating DL tartaric acid, citric acid, mannitol, lactose, sodium chloride, sucrose, and polyethylene glycol with a fluidized bed or by a wet method according to the formulas in Table 6. Preparing granules 2 by granulating sodium bicarbonate, aspartame, lactose, xylitol, essence, sodium carboxymethyl cellulose, and polyethylene glycol with a fluidized bed or by a wet method; Mixing well the granules 1, the granules 2, the cannabinoid solid granules, leucine, essence, carotene, and vitamin C and tableting effervescent tablets each weighing 1 g.

### (2) Second set of preparation process (process 2)

Mixing well the cannabinoid solid granules, DL tartaric acid, citric acid, mannitol, lactose, sodium chloride, sucrose, sodium bicarbonate, aspartame, lactose, xylitol, essence, polyethylene glycol, leucine, essence, carotene, and vitamin C directly according to the formulas in Table 6, and tableting into effervescent tablets each weighing 1 g.

The quality comparison study of effervescent tablets prepared according to different formulas is shown in Table 7:

**Table 7. Quality comparison study of effervescent tablets prepared according to different formulas**

| Formula | Disintegration time min | Uniformity of dispersion | Solution state after effervescence | Content stability |
|---|---|---|---|---|
| 1 | 3 | Acceptable | Clear and transparent | The tablets are stable in content under an accelerated condition for 6 months |
| 2 | 3 | Acceptable | Clear and transparent | The tablets are stable in content under an accelerated condition for 6 months |
| 3 | 5 | Acceptable | Clear and transparent | The tablets are stable in content under an accelerated condition for 6 months |

It can be seen from results in Table 7 that the effervescent tablets prepared according to these drug loading formulas all meet the quality standards of effervescent tablets, and they are quick to dissolve in water, convenient to use and carry, and are capable of increasing the dissolution speed of cannabinoid solid granules. The cannabinoid solution after effervescence can be directly taken, features high bioavailability, and can improve clinical efficacy.

The cannabinoid effervescent tablets have the advantages of being quick to dissolve in water, convenient to use and carry, and capable of increasing the dissolution speed of cannabinoid solid powders.

### Example 5: Preparation of Cannabinoid Enteric Capsules

### 1.Formula:

The cannabinoid solid granules (prepared in Example 2), mannitol, hydroxypropyl methylcellulose, crospolyvinylpyrrolidone, acrylic resin, diacetyl monoglyceride, and talc. The specific formulas are shown in Table 8:

**Table 8. Formulas of cannabinoid enteric capsules**

| Formula | Cannabinoid solid granules% | Mannitol% | Hydroxypropyl methylcellulose% | Crospolyvinylpyrrolidone% | Acrylic resin% | Diacetyl monoglyceride% | Talc% |
|---|---|---|---|---|---|---|---|
| 1 | 20 | 12 | 25 | 25 | 12 | 5 | 1 |
| 2 | 30 | 10 | 24 | 24 | 10 | 2 | 1 |

### 2. Process: (According to the formulas in Table 8)

a. Main drug loading: placing blank pellet cores in a coating pan, dissolving the cannabinoid solid granules and hydroxypropyl methylcellulose, spraying the solution on the blank pellet cores, and drying at 55 °C for 12 h;
b. Protective coating: taking hydroxypropyl methylcellulose and mannitol, adding water, stirring well, spraying the solution on the drug-containing pellet cores, and drying at 55 °C for 12 h;
c. Cohesive coating: taking hydroxypropyl methylcellulose, diacetyl monoglyceride, and talc, adding water, stirring well, spraying the solution on the drug-containing pellet cores, and drying at 55 °C for 12 h;
d. Enteric coating: taking acrylic resin, adding water, stirring well, spraying the solution on the drug-containing pellet cores, and drying at 55 °C for 12 h;
e. Filling: adding talc, and filling capsules.

The quality comparison study of different cannabinoid enteric capsules is shown in Table 9:

**Table 9. Quality comparison of cannabinoid enteric capsules**

| Formula | Difference in filling volume | *In vitro* dissolution rate | Stability |
|---|---|---|---|
| 1 | ±3.0% | Linear release, cumulative release rate 93% | Stable under an accelerated condition for 6 months |
| 2 | ±2.5% | Linear release, cumulative release rate 92% | Stable under an accelerated condition for 6 months |
| 3 | ±2.6% | Linear release, cumulative release rate 92% | Stable under an accelerated condition for 6 months |

It can be seen from results in Table 9 that the cannabinoid sustained-release capsules are up to standard for difference in filling volume, dissolution rate, and stability according to the comparison of different formulas. The cannabinoid is slowly released in intestinal tracts. After entering blood, the cannabinoid water-soluble complex included by micelles and cyclodextrin can slowly release the cannabinoid so that the plasma concentration of the cannabinoid is kept sustained and stable *in vivo,* thus producing a sustained effect for chronic pain.

The cannabinoid enteric capsules can be released in a site-specific way to protect gastric mucosa. Meanwhile, the cannabinoid is slowly released in intestinal tracts. After entering blood, the cannabinoid in the complex of micelles and cyclodextrin can slowly release the cannabinoid so that the plasma concentration of the cannabinoid is kept sustained and stable *in vivo,* thus producing a sustained effect for chronic pain.

### Example 6: Preparation of Cannabinoid Orally Disintegrating Tablets

### 1. Formula:

The cannabinoid inhalant powders (prepared in Example 3) (1-90%), low-substituted hydroxypropylcellulose (1-80%), mannitol (0-80%), microcrystalline cellulose (0-80%), pregelatinized starch (0-80%), colloidal silicon dioxide (0-10%), and magnesium stearate (0-10%).

The specific formulas are shown in Table 10:

**Table 10. Formulas of cannabinoid orally disintegrating tablets**

| Formula | Cannabinoid and a monomer thereof% | Low-substituted hydroxypropylcellulose% | Microcrystalline cellulose% | Mannitol% | Pregelatinized starch% | Colloidal silicon dioxide% | Magnesium stearate% |
|---|---|---|---|---|---|---|---|
| 1 | 10 | 25 | 25 | 18 | 15 | 5 | 2 |
| 2 | 20 | 30 | 20 | 10 | 10 | 5 | 2 |

### 2. Preparation process (According to the formula in Table 10)

### (1) First set of preparation process (process 1)

Mixing well the cannabinoid inhalant powders (prepared in Example 3), low-substituted hydroxypropylcellulose, mannitol, microcrystalline cellulose, pregelatinized starch, colloidal silicon dioxide, and magnesium stearate according to the proportion specified in the formula, and directly tableting into disintegrating tablets directly each weighing 0.5 g.

### (2) Second set of preparation process (process 2)

Granulating the cannabinoid inhalant powders (prepared in Example 3), low-substituted hydroxypropylcellulose, mannitol, microcrystalline cellulose, pregelatinized starch, colloidal silicon dioxide, and magnesium stearate by a wet method or one-step granulating, and tableting into disintegrating tablets each weighing 0.5 g.

The quality comparison study of immediate-release tablets prepared according to different formulas is shown in Table 11:

**Table 11. Quality comparison of cannabinoid orally disintegrating tablets**

| Formula | Appearance | Disintegration time limit s | Difference in tablet weight | Stability |
|---|---|---|---|---|
| 1 | White to brown tablets | 45 | ±2.3% | Stable under an accelerated condition for 6 months |
| 2 | White to brown tablets | 45 | ±2.5% | Stable under an accelerated condition for 6 months |

It can be seen from results in Table 11 that the orally disintegrating tablets prepared according to the formulas are up to standard for appearance, difference in tablet weight, disintegration time limit, difference in tablet weight, and stability. The orally disintegrating tablets can be rapidly dissolved in saliva or rapidly disintegrated in the oral cavity, and they are suitable for patients with tumors, children, the elderly, bed-ridden patients, and seriously disabled patients.

The cannabinoid orally disintegrating tablets can be rapidly dissolved in saliva or rapidly disintegrated in the oral cavity, and they are suitable for patients with tumors, children, the elderly, bed-ridden patients, and seriously disabled patients.

### Example 7: Experimental Results

The water-soluble cannabinoid formulations provided in the examples have the advantages of high encapsulation rate and transfer rate of the effective ingredient, high drug loading, and strong stability. A new self-assembly technology at room temperature is adopted to prevent the active ingredient cannabinoid from degrading and changing color at high temperature; the bioavailability of the active ingredient is high, which can reduce the dose for a single administration. Particularly, the inhalant powders feature high drug loading (10% or more), strong stability (shelf life: 2-3 years), and high *in vitro* deposition rate (the emptying rate can reach 95%, and the effective deposition rate can reach 45%), produce a quick response after inhalation, and can provide sustained and stable plasma concentration.

The above description is only for the purpose of illustrating the preferred examples of the present invention, and is not intended to limit the scope of the present invention. Any modifications, equivalents, and the like made without departing from the spirit and principle of the present invention shall fall in the protection scope of the present invention.

## Claims

1. A water-soluble cannabinoid solid granule, comprising a cannabinoid, an amphiphilic high molecular polymer, and cyclodextrin, wherein a content of the cannabinoid in percentages by weight is 1-40%, a content of the amphiphilic high molecular polymer in percentages by weight is 1-90%, and a content of the cyclodextrin in percentages by weight is 1-90%.

2. The water-soluble cannabinoid solid granule of claim 1, wherein the cannabinoid is selected from one of or a combination of two or more of pure products of CBD, CBDV, CBG CBC, CBN, CBDB, CBE, CBL, and CBND; or
the cannabinoid is a cannabis extract comprising one of or a combination of two or more of CBD, CBDV, CBG CBC, CBN, CBDB, CBE, CBL, and CBND.

3. The water-soluble cannabinoid solid granule of claim 1, wherein the amphiphilic high molecular polymer is selected from one of or a combination of two or more of a poloxamer, a polyethylene glycol-poly(lactic-co-glycolic acid) copolymer, a polyethylene glycol-polycaprolactone block copolymer, a polyethylene glycol-polylactic acid block copolymer, and a polyethylene glycol-polystyrene block copolymer;
preferably, the poloxamer is selected from one of or a combination of two or more of poloxamers 188, 338, 407, 124, 215, and 237;
preferably, a number average molecular weight of the polyethylene glycol-poly(lactic-co-glycolic acid) block copolymer, the polyethylene glycol-polycaprolactone block copolymer, the polyethylene glycol-polylactic acid block copolymer, and the polyethylene glycol-polystyrene block copolymer is 500-50,000, wherein the polyethylene glycol is polyethylene glycol monomethyl ether, and a number average molecular weight of the polyethylene glycol moiety is 200-12,000.

4. The water-soluble cannabinoid solid granule of claim 1, wherein the cyclodextrin is selected from α-cyclodextrin, β-cyclodextrin, γ-cyclodextrin, α-cyclodextrin derivatives, β-cyclodextrin derivatives, and γ-cyclodextrin derivatives.

5. The water-soluble cannabinoid solid granule of claim 4, wherein the cyclodextrin is selected from one of or a combination of two or more of β-cyclodextrin, hydroxypropyl-β-cyclodextrin, methyl-β-cyclodextrin, and propylene oxide-β-cyclodextrin.

6. A water-soluble cannabinoid formulation prepared from the water-soluble cannabinoid solid granule of any one of claims 1-5, wherein a method for preparing the formulation comprises:
(1) adding a cannabinoid, an amphiphilic high molecular polymer, and cyclodextrin to water to prepare a water-soluble complex solution, wherein a mass ratio of materials to water is 1:(1-6);
(2) drying the water-soluble complex solution obtained in step (1) to obtain water-soluble cannabinoid solid granules; and
(3) preparing the cannabinoid solid granules obtained in step (2) into a water-soluble cannabinoid formulation.

7. The water-soluble cannabinoid formulation of claim 6, wherein the water-soluble cannabinoid formulation also comprises a pharmaceutically acceptable lyoprotectant, wherein a content of the lyoprotectant in percentages by weight is 0-20%, and the lyoprotectant is selected from one or more of sodium thiosulfate, sodium bisulfite, sodium sulfite, sodium metabisulfite, vitamin C, lactose, mannitol, sorbitol, EDTA-2Na, trehalose, glucose, xylitol, and maltose.

8. The water-soluble cannabinoid formulation of claim 6, wherein the water-soluble cannabinoid formulation also comprises a pH regulator, wherein a content of the pH regulator in percentages by weight is 0-40%, and the pH regulator is selected from one or more of citric acid, sodium citrate, tartaric acid, sodium tartrate, acetic acid, sodium acetate, sodium dihydrogen phosphate, disodium hydrogen phosphate, hydrochloric acid, lactic acid, and sodium hydroxide.

9. The water-soluble cannabinoid formulation of claim 6, wherein the drying in step (2) is lyophilizing or reduced-pressure drying.

10. The water-soluble cannabinoid formulation of claim 6, wherein the formulation is a solid formulation or a semi-solid formulation;
preferably, the formulation is an oral formulation, an inhalant formulation, or an enteric formulation, wherein
preferably, the formulation is an oral formulation and is preferably selected from an effervescent tablet, a sustained-release tablet, a disintegrating tablet, and a dripping pill;
the formulation is an inhalant formulation, preferably an inhalant powder; or
the formulation is an enteric formulation, preferably an enteric capsule.
